# EUROPEAN PATENT APPLICATION

(11) **EP 4 375 292 A1**
(43) Date of publication of application: **29.05.2024**
(21) Application number: 22846274.3
(22) Date of filing: 21.07.2022
(51) Int. Cl.: C07K 14/705, C07K 16/28, C07K 16/32, A61P 35/00, A61K 35/17

(54) **NOVEL CHIMERIC ANTIGEN RECEPTOR AND IMMUNE CELLS EXPRESSING SAME**

(30) Priority: 21.07.2021 KR 20210096085
(71) Applicant: Korea Research Institute of Bioscience and Biotechnology, Daejeon 34141 (KR)
(72) Inventor: KIM, Tae-Don, Daejeon 34141 (KR); LEE, Sooyun, Daejeon 34141 (KR)
(74) Representative: Berggren Oy
(86) International application number: PCT/KR2022/010729
(87) International publication number: WO 2023/003404

(57) **Abstract**

The present invention relates to: a novel chimeric antigen receptor containing, as an intracellular signaling domain, an intracellular domain of a receptor containing a dead region; and immune cells expressing the chimeric antigen receptor. In environments in which normal cells are present, the immune cells expressing the chimeric antigen receptor according to the present invention exhibit little or no cytotoxicity and cell death of the immune cells is exhibited, thus ensuring the stability of the normal cells. Conversely, in environments in which target cells are present, the immune cells exhibit more potent cytotoxicity than with conventional techniques utilizing a lone chimeric antigen receptor.

## Description

### [Technical Field]

The present invention relates to a novel chimeric antigen receptor comprising an intracellular domain of the receptor containing a death domain as an intracellular signaling domain, and an immune cell expressing the same.

### [Background Art]

A method for treating a cancer have gone through a process of continuous development and change, and have continuously utilized a technology such as a surgery, a chemotherapy, and a radiation therapy to this day. However, there is a problem that these existing methods for treating the cancer are mostly effective only in the early stage when the cancer has not metastasized, and have a high possibility of recurrence later even if the surgery is performed in a stage where metastasis has already progressed. Accordingly, a research has recently continued to develop methods for using an immune response to treat the cancer.

Among them, interest is growing in a cell therapy method that use an immune cell to strengthen it or genetically modify the immune cell and inject it back into a patient. For example, a technology such as a tumor infiltrating lymphocyte (TIL), a chimeric antigen receptor (CAR), and a T-cell receptor (TCR) have been studied. In particular, in the case of the chimeric antigen receptor, which is an artificial receptor designed to deliver antigen specificity to a T-cell or a natural killer cell (NK cell), the receptor consists of an extracellular domain that can activate the immune cell and provide specific immunity by binding to a cancer cell-specific antigen, a transmembrane domain, and an intracellular signaling domain. Also, the T-cell expressing this chimeric antigen receptor was named as a CAR-T-cell (Kershaw et al., Nat. Rev. Immunol., 5(12): 928-940 (2005); Restifo et al., Nat. Rev. Immunol., 12(4): 269-281 (2012)), and the natural killer cell was named as a CAR-NK cell.

The intracellular signaling domain of the chimeric antigen receptor is mainly based on an intracellular signaling domain of CD3zeta, a signaling subunit of the T-cell receptor (the first-generation CAR). Also, it has evolved to add the intracellular signaling domain of co-stimulatory molecules that promotes growth and differentiation of the immune cell in the chimeric antigen receptor. For example, the currently available CAR-T-cell therapies have employed the intracellular signaling domain of CD28 and 4-1BB co-stimulatory molecules, respectively (the second-generation CAR), and thereafter have been attempted to employ the CAR (the third-generation CAR) comprising CD28 and 4-1BB intracellular signaling domains simultaneously (Stegen et al., Nat. Rev. Drug Discov., 14(7): 499-509 (2015)).

However, the therapy such as the CAR-T is effective against a cancer cell, whereas it has caused a side effect due to partially non-specific attack against a healthy tissue in some cases. In order to overcome this, a research has continued to minimize the side effect of attacking the normal cell by increasing antigen recognition ability of the cancer cell. However, there is still a problem in that, once the CAR-T is injected, the toxic T-cell continue to exert its function even after the cancer cell is removed, causing the side effect due to non-specific attack such as cytotoxicity to the normal cell. In addition, since recurrence of the cancer is caused by lack of antigen specificity that is present in the cancer, and diversity and heterogeneity of the antigen, in order to improve anti-cancer efficacy against the recurrence, there is also a need to develop a technology of 'logic-gated CAR advancement', that is, a technology to design the CAR that multi-targets the antigen to improve efficacy against the cancer and ensure safety for the normal cell.

### [Disclosure]

### [Technical Problem]

The purpose of the present invention is to provide a novel chimeric antigen receptor that can amplify cytotoxic activity against a cancer cell without showing cytotoxicity against a normal cell, when expressed in an immune cell.

Further, the purpose of the present invention is to provide a polynucleotide and an expression vector for expressing the chimeric antigen receptor.

Furthermore, the purpose of the present invention is to provide an immune cell that express the chimeric antigen receptor on its surface and has a superior therapeutic effect on a cancer while having safety for a normal cell, so that the immune cell can be useful as a cancer therapy.

Still furthermore, the purpose of the present invention is to provide a pharmaceutical composition for treating a cancer using the immune cell.

### [Technical Solution]

In order to achieve the above purposes, an aspect of the present invention provides a chimeric antigen receptor (CAR) comprising: an extracellular domain (extracellular binding domain) containing an antigen-binding site; a transmembrane domain; and an intracellular signaling domain containing an intracellular domain of the receptor containing a death domain.

Other aspect of the present invention provides a polynucleotide containing a base sequence encoding the chimeric antigen receptor, and an expression vector containing the polynucleotide.

Another aspect of the present invention provides an immune cell that expresses the chimeric antigen receptor as a first chimeric antigen receptor on a surface of the immune cell; and expresses, on a surface of the immune cell, a second chimeric antigen receptor comprising an extracellular domain containing an antigen-binding site that specifically binds to an antigen expressed in a cancer cell, a transmembrane domain, and an intracellular signaling domain.

Still another aspect of the present invention provides a pharmaceutical composition for treating a cancer, comprising the immune cell.

### [Advantage Effects]

A chimeric antigen receptor of the present invention can transmit a signal by an antigen expressed in both of a normal cell and a cancer cell, wherein when transmitting the signal alone, an immune cell expressing the receptor does not show cytotoxicity or show less cytotoxicity. In addition, in case the chimeric antigen receptor binds to the antigen for a long time, it can induce apoptosis of the immune cell, and thus has an advantage of not showing cytotoxicity to the normal cell, not the cancer cell, thereby ensuring stability.

Meanwhile, when the chimeric antigen receptor binds to an antigen expressed in the cancer cell, signal transmittance occurs by acting together with another chimeric antigen receptor that can recognize and bind to a cancer cell-specific antigen, so that the chimeric antigen receptor has characteristic of exerting a synergistic effect capable of exhibiting amplified cytotoxicity compared to in case a single chimeric antigen receptor specific to the cancer cell acts, whereby it can be very useful for treating the cancer.

However, the effects of the present invention are not limited to those mentioned above, and other effects not mentioned will be able to be clearly understood by those skilled in the art from the following description.

### [Description of the Drawings]

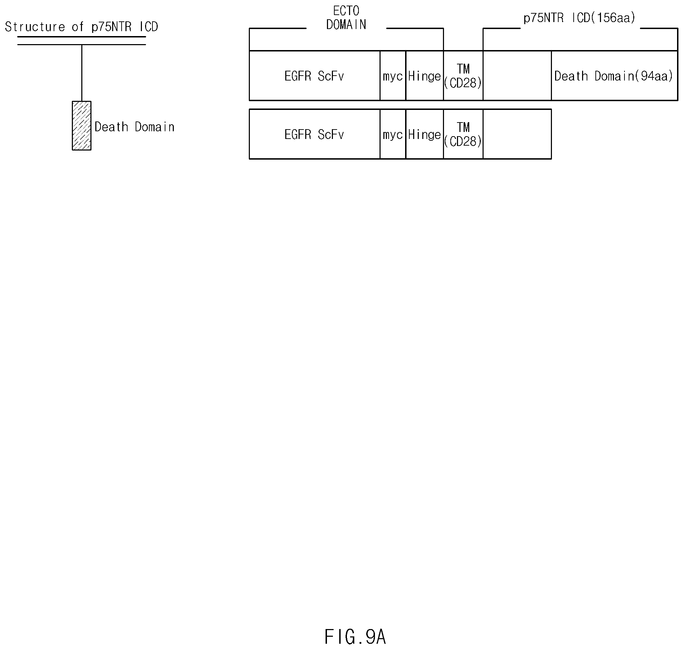

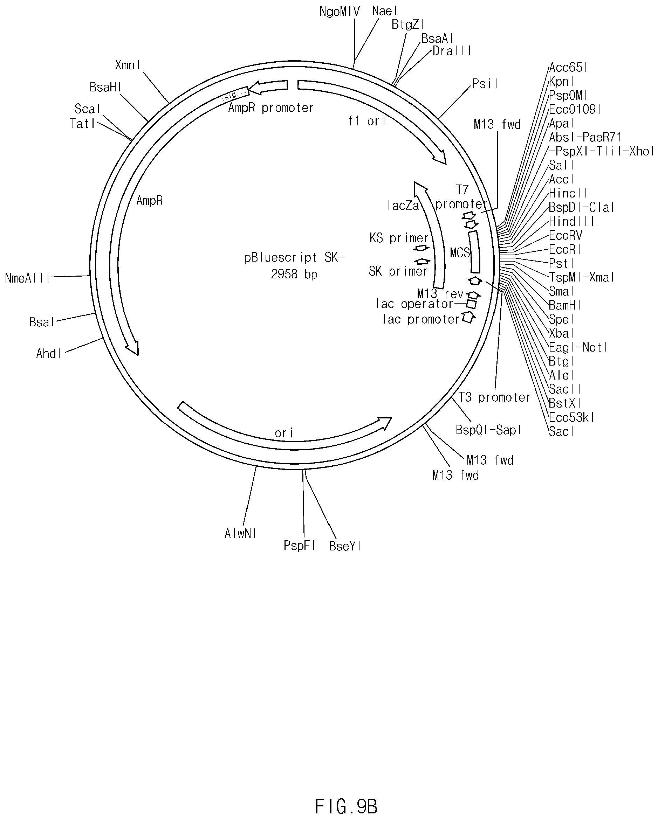

FIG. 1 is a schematic diagram showing a structure of the chimeric antigen receptor of the present invention where EGFR is used as a target antigen, and a diagram showing a structure of a vector for inserting a polynucleotide encoding the chimeric antigen receptor to express it.
FIG. 2 shows the results of confirming expression levels of Myc and zsGreen for a Dual-CAR NK92 cell 1 created by expressing the chimeric antigen receptor of the present invention on a natural killer cell (α-Cot-CAR NK92 (M2)) expressing an anti-cotinine chimeric antigen receptor. In FIG. 2, the 'M₂-EGFR-P75NTR' is a natural killer cell expressing the dual chimeric antigen receptor, and the #1, #2, #3, and #5 are those that separate the natural killer cells expressing the dual chimeric antigen receptor of M₂-EGFR-P75NTR into a single cell.
FIGS. 3A and 3B show the results of confirming expression levels of Myc and zsGreen for a Dual-CAR NK92 cell 2 which was created by expressing the chimeric antigen receptor of the present invention on a natural killer cell (α-EphA2-CAR NK92(79-14)) expressing an anti-EphA2 chimeric antigen receptor.
FIG. 4 shows a comparison of cytotoxicity identified after co-culturing with a breast cancer cell AU565 expressing EGFR using a Dual-CAR NK92 cell 1 and a α-Cot-CAR NK92 (M2) cell, where A is a graph comparing cytotoxicity of all the cells, and B is a graph comparing cytotoxicity of a single cell.
FIGS. 5A and 5B are graphs measuring and comparing occurrence rates of apoptosis over co-culture time after co-culturing with a breast cancer cell AU565 expressing EGFR, using a Dual-CAR NK92 cell 1 and a α-Cot-CAR NK92 (M2) cell.
FIG. 6 shows a comparison of cytotoxicity identified after co-culturing a breast cancer cell AU565 expressing EGFR and HER2 together with a HER2-Cot conjugate, using a Dual-CAR NK92 cell 1 and a α-Cot-CAR NK92 (M2) cell, where A is a graph comparing cytotoxicity of all the cells, and B is a graph comparing cytotoxicity of a single cell.
FIG. 7 shows a comparison of cytotoxicity identified after co-culturing with a breast cancer cell MDA-MB-231 expressing both EGFR and EphA2, using a Dual-CAR NK92 cell 2 and a α-Epha2-CAR NK92(79-14) cell.
FIG. 8 shows a graph measuring and comparing occurrence rates of apoptosis after co-culturing with a breast cancer cell MDA-MB-231 expressing both EGFR and EphA2 followed by curing for 17 hours, using a Dual-CAR NK92 cell 2 and a α-Epha2-CAR NK92(79-14) cell.
Each of FIGS. 9A and 9B is a schematic diagram showing a structure of a chimeric antigen receptor (p75^{NTR} DDD) from which a death domain of the intracellular domain of p75^{NTR} has been removed within the chimeric antigen receptor of the present invention, and a diagram showing a structure of a vector for inserting a polynucleotide encoding the chimeric antigen receptor to express it.

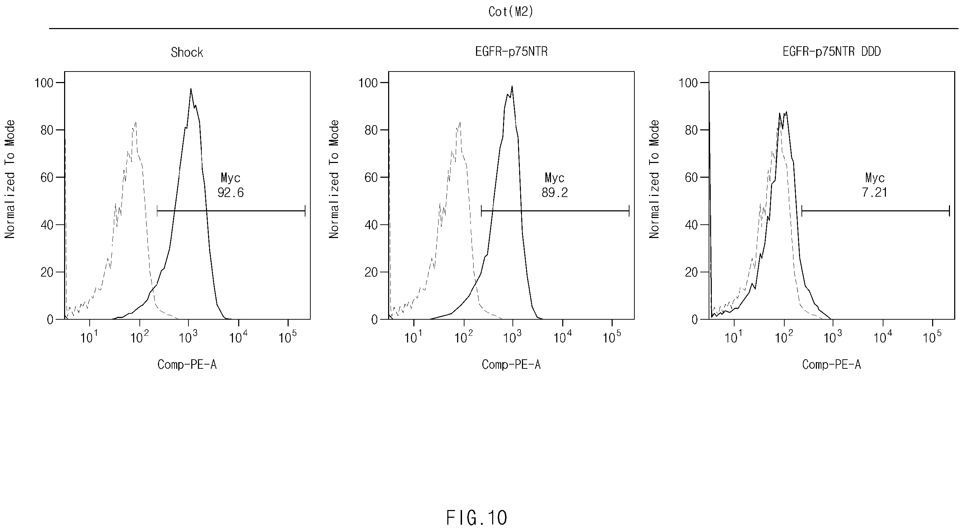

FIG. 10 shows the results of confirming an expression level of Myc for a Dual-CAR NK92 cell created by expressing a chimeric antigen receptor (p75^{NTR} DDD), from which a death domain of the intracellular domain of p75^{NTR} has been removed within the chimeric antigen receptor of the present invention, on a natural killer cell (α-Cot-CAR NK92 (M2)) expressing an anti-cotinine chimeric antigen receptor.

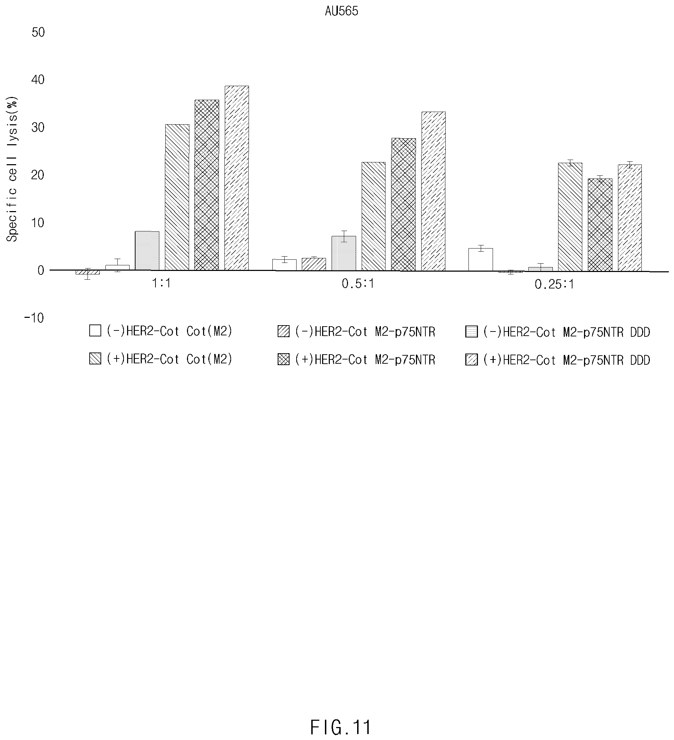

FIG. 11 shows a comparison of cytotoxicity identified after co-culturing a breast cancer cell AU565 expressing EGFR and HER2 together with a HER2-Cot conjugate, using a Dual-CAR NK92 cell made by expressing a chimeric antigen receptor (p75^{NTR} DDD) from which a death domain of the intracellular domain of p75^{NTR} has been removed, a Dual-CAR NK92 Cell 1 and a α-Cot-CAR NK92 (M2) cell.

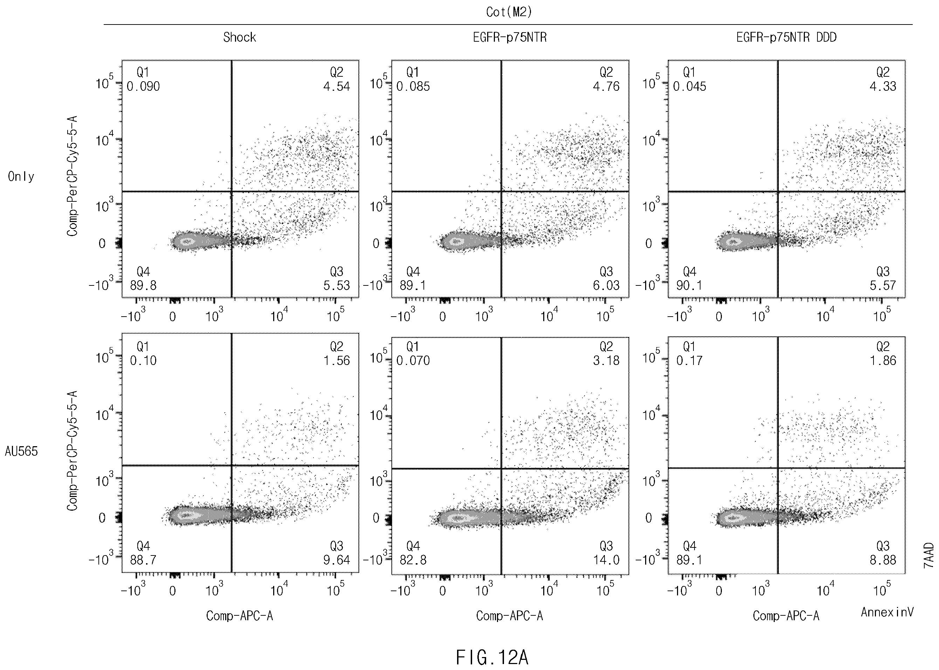

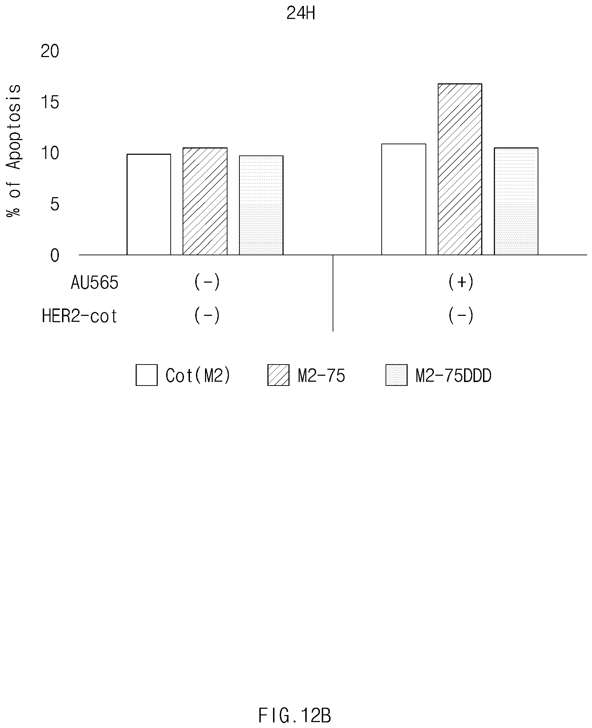

FIGS. 12A and 12B are graphs measuring and comparing occurrence rates of apoptosis over co-culture time after co-culturing with a breast cancer cell AU565 expressing EGFR, using a Dual-CAR NK92 cell made by expressing a chimeric antigen receptor (p75^{NTR} DDD) from which a death domain of the intracellular domain of p75^{NTR} has been removed, a Dual-CAR NK92 Cell 1 and a α-Cot-CAR NK92 (M2) cell.

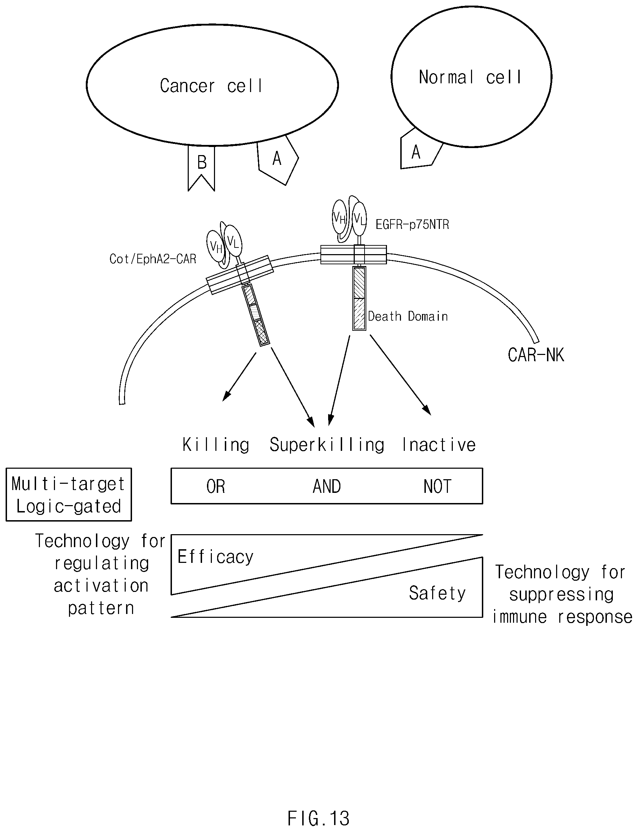

FIG. 13 is a diagram schematically showing the principle of operation of a Dual-CAR NK92, which expresses the chimeric antigen receptor of the present invention.

### [Mode for Invention]

Hereinafter, the present invention will be described in detail.

### 1. Novel chimeric antigen receptor (CAR), and polynucleotide and expression vector for expressing same

An aspect of the present invention provides a novel chimeric antigen receptor that exhibits safety for a normal cell while amplifying cytotoxicity along with other chimeric antigen receptors against a cancer cell.

In the present invention, the term "chimeric antigen receptor (CAR)" is a synthetic complex designed to induce an immune response when it recognizes and binds to a target antigen and a cell expressing an antigen. The chimeric antigen receptor may comprise an extracellular domain (extracellular binding domain), a transmembrane domain, and an intracellular signaling domain. The chimeric antigen receptor is expressed on a surface of the immune cell, and recognizes and binds to a specific antigen such as an antigen specifically expressed on a surface of the cancer cell, through an antigen-binding site contained in the extracellular domain, so that the chimeric antigen receptor can transmit a signal within the immune cell to change activity of the immune cell, thereby causing the immune response by targeting only the specific antigen.

The chimeric antigen receptor of the present invention comprises an extracellular domain (extracellular binding domain) containing the antigen-binding site; a transmembrane domain; and an intracellular signaling domain containing an intracellular domain of the receptor containing a death domain.

The receptor containing the death domain refers to a receptor molecule that has an amino acid sequence involved in apoptosis-inducing activity as the intracellular domain. The receptor containing the death domain includes Fas, TNFRI, p75^{NTR}, DR3, DR4, DR5, DR6, EDAR, etc., but are not particularly limited thereto. In particular, among them, the p75^{NTR} (p75 neurotrophin receptor) is a type of a neurotrophin receptor related to differentiation of a nerve cell and is known as a type of a low-affinity nerve growth factor receptor (LNGFR). The p75^{NTR} consists of an extracellular domain, a transmembrane domain, and an intracellular domain, and the present invention employs the intracellular domain of p75^{NTR}. The intracellular domain of p75^{NTR} may be a global-like domain, and may specifically be a protein containing the amino acid sequence of SEQ ID NO: 1, without being limited thereto. The amino acid sequences described above may include variants having different sequences due to deletion, insertion, substitution, or combination of the amino acid residues, to the extent that they do not affect a structure, function, activity, etc. of the protein containing them. In addition, the amino acid sequences may include amino acids that have undergone common modifications known in the art, wherein the modification of the amino acid may include, for example, phosphorylation, sulfation, acrylation, glycosylation, methylation, famesylation, etc. The intracellular domain of p75^{NTR} of the present invention not only contains the amino acid sequence described above, but also contains an amino acid sequence substantially identical thereto or a variant thereof. The substantially identical amino acid sequence may mean that the amino acid sequence described above is an amino acid sequence having a homology of 90 % or more, 91 % or more, 92 % or more, 93 % or more, 94 % or more, 95 % or more, 96 % or more, 97 % or more, 98 % or more, 99 % or more, or 99.5 % or more, but is not limited thereto.

When the chimeric antigen receptor of the present invention is expressed on a surface of the immune cell, a target antigen binds to the receptor and signal transmission occurs alone by the intracellular domain of p75^{NTR}, which may lead to a reduction in cytotoxicity of the immune cell and/or promotion of apoptosis of the immune cell. The reduction in cytotoxicity may mean that the immune cell does not exhibit cytotoxicity, or that cytotoxicity exhibited by the immune cell is less than that of an immune cell in the absence of an antigen, or that the immune cell exhibits less cytotoxicity compared to an immune cell activated by the antigen. The promotion of apoptosis of the immune cell may lead to more apoptosis than that of an immune cell in the absence of the antigen. The occurrence of signal transmission alone may mean that the signal transmission does not occur by other chimeric antigen receptor, and more specifically may mean that the signal transmission does not occur through a chimeric antigen receptor generating a signal that promotes cytotoxicity of the immune cell against the cancer cell, but occurs only though the chimeric antigen receptor of the present invention.

The extracellular domain contains an antigen-binding site that specifically binds to a desired target antigen, but is not limited to its type. The extracellular domain can be applied without any limitation as long as it has a structure that can specifically recognize and bind to a biological molecule such as a cell surface receptor, a tumor protein, a lipid, a polysaccharide, or other cell surface target molecule. For example, the extracellular domain may be a protein, a polypeptide, or a variant thereof which has the characteristics described above. The term "specifically bind to" may mean binding to another molecule with a binding affinity that is greater than background binding, and, for example, may mean that the extracellular domain binds to a target antigen with an affinity of about 10⁻⁵ M or more for the target antigen, or a Ka (an equilibrium dissociation constant of specific binding interaction having the unit of 11M). The affinity may mean that the equilibrium dissociation constant (Ka) of specific binding interaction having the unit of M is 10⁻⁵ M to 10⁻¹³ M or less than this range.

The antigen-binding site may specifically bind to an antigen expressed in both of the cancer cell and the normal cell. Concretely, the antigen expressed in both of the cancer cell and the normal cell may not be a cancer cell-specific antigen, that is, it may not be a cancer-specific marker used to prevent, treat, improve, or diagnose a cancer. Therefore, the chimeric antigen receptor of the present invention can recognize and bind to both of the cancer cell and the normal cell, thereby generating signal transmission in the immune cell expressing the chimeric antigen receptor. The antigen-binding site may, for example, specifically bind to EGFR, but is not limited thereto.

The antigen-binding site may be an antibody or an antigen binding fragment thereof. More specifically, the antigen-binding site may be a single chain variable fragment (scFv) of the antibody. Concretely, the antigen-binding site may be a single chain variable fragment of the antibody that specifically binds to EGFR, and more specifically, may include an amino acid sequence of SEQ ID NO: 3.

The antibody refers to an immunoglobulin molecule that is immunologically reactive by specifically binding to an epitope of the antigen. The antibody may include a monoclonal antibody, a polyclonal antibody, an antibody with a full-length chain structure (full-length antibody), a functional fragment with at least antigen-binding function (antigen-binding fragment), and a recombinant antibody. Concretely, the antibody of the present invention may be the monoclonal antibody or an antigen-binding fragment thereof. The monoclonal antibody refers to an antibody molecule of single molecular composition obtained from a substantially identical antibody population, and such monoclonal antibody exhibits a single binding specificity and affinity for a specific epitope. The full-length antibody has a structure of two full-length light chains and two full-length heavy chains, and each light chain may be connected to the heavy chain through a disulfide bond. The antibody may include heavy chain (HC) and light chain (LC) polypeptides, and the heavy and light chains may include a variable region and a constant region.

The constant region is a site that mediates binding of the antibody to various cells of an immune system (T-cell, etc.) or a host tissue containing components of a complement system, etc. The constant region has the same function regardless of a type of the antigen if it is the same type of an antibody derived from the same species, and the amino acid sequence forming it is also the same or has a high degree of similarity for each antibody. The constant region may be divided into a heavy chain constant region (which may be abbreviated as CH) and a light chain constant region (which may be abbreviated as CL). The heavy chain constant region has gamma (γ), mu (µ), alpha (α), delta (δ) and/or epsilon (ε) types, and is subclassed as gamma 1 (γ1), gamma 2 (γ2), gamma 3 (γ3), gamma 4 (γ4), alpha 1 (α1) and/or alpha 2 (α2). The light chain constant region has kappa (κ) and lambda (λ) types. IgG is subclassed as IgG1, IgG2, IgG3, and IgG4.

The variable region is an antibody site that has specificity for an antigen and may be divided into a heavy chain variable region (which may be abbreviated as VH) and a light chain variable region (which may be abbreviated as VL). The variable region may include three complementary-determining regions (CDRs) and four framework regions (FRs). The CDR may be a ring-shaped site involved in antigen recognition, and specificity to the antigen may be determined depending on an amino acid sequence of the CDR. The CDR may be referred to as CDR1, CDR2, and CDR3 in its order. Depending on whether the CDR is of the heavy chain or light chain polypeptide, the heavy chain variable region may be referred to as CDR-H1, CDR-H2, and CDR-H3, and the light chain variable region may be referred to as CDR-L1, CDR-L2, and CDR-L3. Likewise, the FR may be referred to as FR-H1, FR-H2, FR-H3, and FR-H4 for the heavy chain variable region, and referred to as FR-L1, FR-L2, FR-L3, and FR-L4 for the light chain variable region.

The antigen-binding fragment refers to any fragment of an antibody that retains antigen-binding function of the antibody. The antigen-binding fragment may be referred to interchangeably with terms such as "fragment" or "antibody fragment," and the antigen-binding fragment may be Fab, Fab', F(ab')₂, Fv, etc., but is not limited thereto.

The Fab is a structure that includes a variable region of the light and heavy chains, a constant region of the light chain, and a first constant region (CH1 domain) of the heavy chain, and has one antigen-binding site. The Fab' differs from the Fab in that it has a hinge region containing one or more cysteine residues at a C-end of the heavy chain CH1 domain. The F(ab')₂ is generated when the cysteine residue in the hinge region of Fab' forms a disulfide bond. The Fv refers to a minimum antibody fragment containing only the heavy chain variable region and the light chain variable region. A dual-chain variable fragment connects the heavy chain variable region and the light chain variable region by a noncovalent bond. A single chain variable fragment (scFv) generally connects the heavy chain variable region and the light chain variable region through a peptide linker by a covalent bond or connects them at the C-end directly to form a dimer like the dual- chain variable fragment. The antigen-binding fragment can be produced by using a proteolytic enzyme (for example, the Fab can be obtained by restriction digestion of the entire antibody with papain, and the F(ab')₂ fragment can be obtained by digestion with pepsin), or can be produced through a genetic recombination technology, but is not limited thereto.

The linker may be a peptide linker and may have a length of about 10 to 25 amino acids. For example, the linker may include a hydrophilic amino acid such as glycine (G) and/or serine (S). The linker may include, for example, (GS)n, (GGS)n, (GSGGS)n or (GnS)m (n and m are 1 to 10, respectively.), and may include, for example, (GnS)m( n and m are 1 to 10, respectively.), but are not limited thereto.

The extracellular domain may further include at least one selected from the group consisting of a hinge domain and a spacer domain. The antigen-binding site of the extracellular domain may be connected to the transmembrane domain through the hinge domain and/or the spacer domain.

The hinge domain is a portion allowing the antigen-binding site to be physically spaced apart from a surface of the immune cell expressing the chimeric antigen receptor so as to enable appropriate contact of the cell/cell, appropriate binding of the antigen/antigen-binding site, and appropriate activation of the chimeric antigen receptor, and can play an important role in determining position of the extracellular domain. The chimeric antigen receptor may comprise one or more hinge domains between the extracellular domain and the transmembrane domain. The hinge domain may be derived from natural, synthetic, semi-synthetic or recombinant sources. The hinge domain may comprise the amino acid sequence of a naturally occurring immunoglobulin hinge region or a modified immunoglobulin hinge region. The modified hinge region refers to (a) a naturally occurring hinge region having the amino acid modification of up to 30% (e.g., amino acid substitution or deletion of up to 25%, up to 20%, up to 15%, up to 10%, or up to 5%), (b) a part of the naturally occurring hinge region of at least 10 amino acids (e.g., at least 12, 13, 14, or 15 amino acids) in length, which has the amino acid modification of up to 30% (e.g., amino acid substitution or deletion of up to 25%, up to 20%, up to 15%, up to 10%, or up to 5%), or (c) a part of the naturally occurring hinge region containing a core hinge region (which may be 4, 5, 6, 7, 8, 9, 10, 11, 12, 13, 14 or 15, or at least 4, 5, 6, 7, 8, 9, 10, 11, 12, 13, 14, or 15 amino acids in length). In a certain embodiment, one or more cysteine residues in the naturally occurring immunoglobulin hinge region may be substituted with one or more other amino acid residues (e.g., one or more serine residues). The modified immunoglobulin hinge region may alternatively or additionally have another amino acid residue, for example, a proline residue of the wild-type immunoglobulin hinge region substituted with a cysteine. The hinge domain may be a hinge region derived from the extracellular domain of a type 1 membrane protein such as CD8, CD4, CD28, and CD7, but any hinge domain can be used without limitation as long as the antigen-binding site, the transmembrane domain, and the intracellular signaling domain can be connected between the cell membranes. Additionally, the hinge domain may be a wild-type hinge region from these molecules or may be modified.

The spacer domain may be referred to as a linking domain, and may include, for example, a hinge domain derived from CD28 and/or a hinge domain derived from CD8. The spacer domain may include all or a part of the hinge domain derived from CD28 and/or the hinge domain derived from CD8.

The hinge domain and/or the spacer domain may be at least one selected from the group consisting of a Myc epitope, a CD8 hinge domain, and an Fc, and may specifically include the Myc epitope and the CD8 hinge domain. More specifically, the Myc epitope may include an amino acid sequence of SEQ ID NO: 5, and the CD8 hinge domain may include an amino acid sequence of SEQ ID NO: 7.

The transmembrane domain refers to a part of the domain that plays a role to connect and fuse the extracellular domain and the intracellular signaling domain and anchor the chimeric antigen receptor to a plasma membrane of the immune cell. The transmembrane domain may be derived from natural, synthetic, semi-synthetic or recombinant sources. The transmembrane domain may be any one selected from the group consisting of alpha (α), beta (β) or zeta (ζ) chain of the T-cell receptor (TCR), CD28, CD3 epsilon (ε), CD45, CD4, CD5, CD8, CD9, CD16, CD22, CD33, CD37, CD64, CD80, CD86, CD134, CD137, and CD154, but is not limited thereto.

The transmembrane domain may be attached to the extracellular domain through a linker. For example, the linker may be a short oligopeptide or polypeptide linker of 2 to 10 amino acids in length, for example, a glycine (G)-serine (S) doublet, but is not limited thereto.

A specific embodiment of the present invention designed a chimeric antigen receptor in which a single chain variable fragment (scFv) and a CD28 were linked with Myc and a hinge domain, wherein the extracellular domain contains the single chain variable fragment of an antibody that specifically binds to EGFR, and the transmembrane domain contains the CD28. Then, the chimeric antigen receptor of the present invention was prepared by linking the intracellular domain of p75^{NTR}, one of the receptors comprising a death domain, to the transmembrane domain with the intracellular signaling domain.

As described above, the chimeric antigen receptor of the present invention can induce reduction in cytotoxicity of the immune cell and/or promotion in apoptosis of the immune cell when signal transmission occurs alone by the intracellular domain of the receptor containing the death domain, such as p75^{NTR}. Therefore, when the normal cell is present and the antigen-binding site of the chimeric antigen receptor recognizes and binds to an antigen expressed from the normal cell, reduction in cytotoxicity of the immune cell and/or promotion in apoptosis of the immune cell can be induced by the signal transmission, whereby the chimeric antigen receptor of the present invention can be usefully used as a chimeric antigen receptor that does not have cytotoxicity against the normal cell and shows stability.

Another aspect of the present invention provides a polynucleotide and an expression vectors for expressing the chimeric antigen receptor.

The polynucleotide includes a base sequence encoding the chimeric antigen receptor.

In the present invention, the term "polynucleotide" comprehensively includes DNA (gDNA and cDNA) and RNA molecules, and the nucleotide, which is a basic structural unit, includes not only a natural nucleotide but also an analogue having a sugar or a base site modified.

Encoding the chimeric antigen receptor means that the polynucleotide encodes genetic information that can synthesize a protein having the amino acid sequence of the chimeric antigen receptor of the present invention through a typical protein expression process such as transcription and translation. In this case, the scope of the present invention may include even polynucleotide that encodes not only a protein having an amino acid sequence completely identical to the chimeric antigen receptor, but also a protein having an amino acid sequence substantially identical to the protein as described above or a protein having the same and/or similar activity as the protein.

Specifically, the polynucleotide of the present invention may comprise the intracellular domain of the receptor containing the death domain, and for example, may comprise a base sequence encoding the intracellular domain of p75^{NTR}, and more specifically may comprise a base sequence of SEQ ID NO: 2.

Further, the polynucleotide of the present invention may comprise the intracellular domain of the receptor containing the death domain, and for example, may comprise the base sequence encoding the intracellular domain of p75^{NTR} as well as the base sequence that encodes the transmembrane domain and/or the extracellular domain linked thereto.

The description of the chimeric antigen receptor such as the intracellular signaling domain containing the intracellular domain of the receptor containing the death domain, such as p75^{NTR}, the transmembrane domain, and the extracellular domain is the same as that described previously.

The polynucleotide may comprise a base sequence of SEQ ID NO: 13. A specific embodiment of the present invention designed a chimeric antigen receptor in which a single chain variable fragment (scFv) and a CD28 were linked with Myc and a hinge domain, and the intracellular domain of p75^{NTR}, one of the receptors containing the death domain was linked to the transmembrane domain with the intracellular signaling domain, wherein the extracellular domain contains the single chain variable fragment of an antibody that specifically binds to EGFR, and the transmembrane domain contains the CD28. To this end, the polynucleotide comprising the base sequence of SEQ ID NO: 13 was prepared to express the chimeric antigen receptor.

The polynucleotide of the present invention may comprise a base sequence substantially identical to the base sequences listed above. The substantially identical base sequence includes, for example, a base sequence where the same amino acid can be synthesized when transcribed and translated, and may be a base sequence having a homology of 90 % or more, 91 % or more, 92 % or more, 93 % or more, 94 % or more, 95 % or more, 96 % or more, 97 % or more, 98 % or more, 99 % or more, or 99.5 % or more with the base sequences listed above, but is not limited thereto.

The polynucleotide encoding the chimeric antigen receptor may comprise an optimized base sequence depending on the type of an organism into which the receptor is intended to be introduced and expressed, and an expression system such as transcription and translation of the organism. This is caused by degeneracy of a codon, and thus various combinations of nucleotide sequences that can encode the expressed protein may exist, all of which are fallen within the scope of the present invention. Modification of the polynucleotide according to the codon optimization may be determined depending on the type of the organism to which the chimeric antigen receptor of the present invention is intended to be expressed and applied. For example, the polynucleotide of the present invention may be a modified polynucleotide to optimally select the codon for mammals and primates, and more specifically may be optimized and modified to be suitable for expression and function from a human.

An expression vector of the present invention comprises the above polynucleotide.

Since the polynucleotide contains a base sequence encoding the chimeric antigen receptor of the present invention, the expression vector comprising it may be used to express and produce the chimeric antigen receptor, and may serve to transfer the polynucleotide to a specific cell or an organism such that the chimeric antigen receptor can be expressed, or can be used to preserve and store the polynucleotide.

The expression vector may be constructed using a prokaryotic cell or an eukaryotic cell as a host.

For example, in case the expression vector uses the prokaryotic cell as the host, it generally includes a strong promoter capable of advancing transcription (e.g., tac promoter, lac promoter, lacUV5 promoter, lpp promoter, pLλ promoter, pRλ promoter, rac5 promoter, amp promoter, recA promoter, SP6 promoter, trp promoter, and T7 promoter, etc.), a ribosome-binding site for initiating translation, and a transcription/translation termination sequence. In case E. coli (e.g., HB101, BL21, DH5α, etc.) is used as the host cell, a promoter and operator sites of the E coli tryptophan biosynthetic pathway (Yanofsky, C, J Bacteriol, (1984) 158:1018-1024), and a left-handed promoter of phage λ (pLλ promoter, Herskowitz, I and Hagen, D, Ann Rev Genet, (1980) 14:399-445) may be used as a regulatory site. In case Bacillus bacteria are used as the host cell, a promoter of the toxin protein gene of Bacillus thuringiensis (Appl Environ Microbiol (1998) 64:3932-3938; Mol Gen Genet (1996) 250:734-741) or any promoter that can be expressed in Bacillus bacteria may be used as the regulatory site. The expression vector may be produced by engineering a plasmid (e.g., pCL, pSC101, pGV1106, pACYC177, ColE1, pKT230, pME290, pBR322, pUC8/9, pUC6, pBD9, pHC79, pIJ61, pLAFR1, pHV14, pGEX series, pET series, and pUC19, etc.), a phage (e.g., λgt4·λB, λ-Charon, λΔz1, and M13, etc.), or a virus (e.g., SV40, etc.), which are often used in the art.

In case the expression vector uses the eukaryotic cell as the host, a promoter derived from a genome of a mammalian cell (e.g., metallothionine promoter, β-actin promoter, human hemoglobin promoter, and human muscle creatine promoter) or a promoter derived from a mammalian virus (e.g., adenovirus late promoter, vaccinia virus 75K promoter, SV40 promoter, cytomegalovirus (CMV) promoter, tk promoter of HSV, mouse mammary tumor virus (MMTV) promoter, LTR promoter of HIV, promoter of Moloney virus, promoter of Epstein-Barr virus (EBV), and promoter of Roux Sarcoma virus (RSV)) may be used. The expression vector may generally have a polyadenylation sequence as a transcription termination sequence. The expression vector may have the CMV promoter.

Further, the expression vector may be fused with other sequences to facilitate purification of the antibody expressed therefrom. The sequences to be fused include, for example, glutathione S-transferase (Pharmacia, USA), maltose binding protein (NEB, USA), FLAG (IBI, USA), 6x His (hexahistidine; Quiagen, USA), etc. In addition, since the protein expressed by the expression vector of the present invention is a humanized antibody or an antigen-binding fragment thereof, considering its characteristics, the expressed protein can be easily purified through a protein A column or the like without additional sequences for purification.

The expression vector may contain an antibiotic resistance gene commonly used as a selection marker in the art, and may contain, for example, a resistance gene for ampicillin, gentamicin, carbenicillin, chloramphenicol, streptomycin, kanamycin, geneticin, neomycin, and tetracycline.

### 2. Immune cell expressing dual chimeric antigen receptor (Dual-CAR)

Another aspect of the present invention provides an immune cell that exhibits enhanced cytotoxicity against a cancer cell while not showing cytotoxicity against a normal cell.

The immune cell expresses the chimeric antigen receptor of the present invention described above as a first chimeric antigen receptor on a surface of the immune cell; and expresses, on a surface of the immune cell, a second chimeric antigen receptor comprising an extracellular domain containing an antigen-binding site that specifically binds to an antigen expressed in the cancer cell, a transmembrane domain, and an intracellular signaling domain.

The first chimeric antigen receptor the same as that described for the chimeric antigen receptor in "1. Novel chimeric antigen receptor (CAR), and polynucleotide and expression vector for expressing same" on the above. Specifically, the first chimeric antigen receptor may include an antigen-binding site capable of specifically binding to an antigen expressed in both of the normal cell and the cancer cell, and an antigen to which the first chimeric antigen receptor binds may be different from an antigen to which the second chimeric antigen receptor binds.

The second chimeric antigen receptor can be described as follows.

The second chimeric antigen receptor is typically used in the art to target a cancer and activate the immune cell to induce an immune response, so any second chimeric antigen receptor that is available as a cancer therapy can be used regardless of a type thereof.

Specifically, the extracellular domain of the second chimeric antigen receptor contains an antigen-binding site that specifically binds to an antigen expressed in the cancer cell. Specifically, the antigen may be a cancer cell-specific antigen, which may be a cancer-specific marker used to prevent, treat, improve, or diagnose the cancer. In other words, the cancer cell-specific antigen may be an antigen statistically expressed at a higher frequency in the cancer cell than in the normal cell significantly, and therefore, the second chimeric antigen receptor can specifically recognize and bind to the cancer cell rather than the normal cell.

The antigen-binding site of the second chimeric antigen receptor may be applied without limitation as long as it can specifically bind to the cancer cell-specific antigen, and may specifically bind to, for example, HER2, EphA2, ErbB3, IL-13Rα2, DLK1, B7H3, PD-L1, GPC3, CEACAM6, CD5, etc.

The extracellular domain of the second chimeric antigen receptor is the same as the extracellular domain of the first chimeric antigen receptor described in "1. Novel Chimeric Antigen Receptor (CAR), and polynucleotide and expression vector for expressing same", except for the description of the type of the antigen to which the antigen-binding site specifically binds. For example, the antigen-binding site of the second chimeric antigen receptor may be a single chain variable fragment of an antibody.

The description of the transmembrane domain of the second chimeric antigen receptor is also same as that of the transmembrane domain of the first chimeric antigen receptor in "1. Novel chimeric antigen receptor (CAR), and polynucleotide and expression vector for expressing same."

The intracellular signaling domain of the second chimeric antigen receptor is responsible for transmitting a signal generated by combination of the chimeric antigen receptor and the antigen to the inside of the immune cell so as to trigger function of the immune cell (e.g., activation including release of a cytotoxic factor against a target-cell to which the second chimeric antigen receptor and the antigen are bound, cytokine production, proliferation and cytotoxic activity, or other cellular response induced by binding of the antigen). The intracellular signaling domain may be part of a protein that transmits a functional signal and directs the cell to perform a specific function.

The intracellular signaling domain of the second chimeric antigen receptor may be an intracellular signaling domain that was used during the development of the chimeric antigen receptor previously. Specifically, it may include only CD3ζ, which was used in the first-generation CAR (chimeric antigen receptor). Also, as used in the second-generation CAR, it may include a form combining a co-stimulatory domain (CD28 or CD137/4-1BB) and CD3ζ to improve responsiveness to the immune cell. Further, it may include two or more of the co-stimulatory domains, as used in the third-generation CAR, wherein the co-stimulatory domain may be combined with 4-1BB, CD28 or OX40, etc. to achieve expansion and persistence of the CAR-containing immune cell in vivo. Furthermore, as used in the fourth-generation CAR, the intracellular signaling domain may allow additional expression of a CAR-based immune protein of cytokine such as IL-12 or IL-15, including an additional gene encoding the cytokine, and as used in the fifth-generation CAR, it may additionally include an interleukin receptor chain, for example, IL-2Rβ, to strengthen the immune cell.

More specifically, activation of the immune cell by the intracellular signaling domain may be mediated by two different classes of the intracellular signaling domains. For example, the activation of the immune cell may be mediated by a co-stimulatory signaling domain that acts in an antigen-independent manner to provide a primary signaling domain that initiate antigen-dependent primary activation, and a secondary signal. Accordingly, the intracellular signaling domain may include the primary signaling domain and the co-stimulatory signaling domain.

The primary signaling domain refers to a signaling domain that regulates the activation of the immune cell in a stimulatory or inhibitory manner. The primary signaling domain that acts in the stimulatory manner may contain a signaling motif known as the immunoreceptor tyrosine-based activation motif or ITAM. The ITAM containing the primary signaling domain may include, but is not limited to, TCRζ, FcRγ, FcRβ, CD3γ, CD3δ, CD3ε, CD3ζ, CD5, CD22, CD79a, CD79b, CD66d, etc. More specifically, the primary signaling domain may be CD3ζ(zeta), but is not limited thereto.

The co-stimulatory signaling domain refers to the intracellular signaling domain of a co-stimulatory molecule. The co-stimulatory signaling domain may include a co-stimulatory signaling domain selected from the group consisting of ligands that specifically bind to CD2, CD7, CD27, CD28, CD30, CD40, 4-1BB (CD137), OX40 (CD134), CDS, ICAM-1, ICOS (CD278), LFA-1 (CD11a/CD18), GITR, MyD88, DAP10, DAP12, PD-1, LIGHT, NKG2C, B7-H3, CD83, etc., but is not limited thereto. Specifically, the co-stimulatory molecule may be DAP 10, without being limited thereto.

According to an embodiment of the present invention, the second chimeric antigen receptor can allow a natural killer cell to exhibit an effect of killing the cancer cell with high activity by using DAP10 and CD3ζ as the intracellular signaling domain.

The second chimeric antigen receptor may include two or more intracellular signaling domains. In the case of including the two or more intracellular signaling domains, the intracellular signaling domain may be linked to each other in series. Alternatively, it may be linked through a polypeptide linker consisting of 2 to 10 amino acids, and the linker sequence may be, for example, a glycine-serine continuous sequence. The linker may include, for example, (GS)n, (GGS)n, (GSGGS)n, or (GnS)m (n, m are 1 to 10, respectively.), for example (GnS)m(n and m are 1 to 10, respectively.), but are not limited thereto.

The second chimeric antigen receptor may further comprise an immune function promoting factor for the immune cell. For example, the immune function promoting factor for the immune cell may be an interleukin signal sequence. The interleukin signal sequence may be characterized by inducing expression of interleukin (IL)-12, IL-8, IL-2, etc., but is not limited thereto. Also, in case the immune cell is a T-cell, the immune function promoting factor may be IL-7, CCL19, etc., but is not limited thereto.

The immune cell can be used without any limitation as long as it is a cell that can induce immunity and cause the desired therapeutic effect, and, for example, may be any one selected from the group consisting of a natural killer cell (NK cell), a T-cell, a natural killer T-cell (NKT-cell), a cytokine-induced killer cell (CIK), a macrophage, and a dendritic cell, but is not limited thereto. Therefore, the immune cell expressing the first chimeric antigen receptor and the second chimeric antigen receptor according to the present invention on a surface of the cell may include a CAR-NK cell (Chimeric Antigen Receptor Natural Killer Cell), a CAR-T-cell (Chimeric Antigen Receptor T-cell), a CAR-NKT-cell (Chimeric Antigen Receptor Natural killer T-cell), a CAR-macrophage (Chimeric Antigen Receptor Macrophage), etc.

The T-cell may be, but is not limited to, a cytotoxic T lymphocyte (CTL), a tumor infiltrating lymphocyte (TIL), a T-cell isolated from a peripheral blood mononuclear cell (PBMC), etc.

The CAR-NK cell refer to a cell in which the chimeric antigen receptor has been introduced into the natural killer cell. The CAR-NK cell has the advantage of being available as a general-purpose therapy by being able to target various cancer cells as well as solving through switching of the on/off reaction a problem due to persistent toxicity of a cancer immunotherapy when the existing CAR-T treatment based on the T-cell was used, a risk of the autoimmune disease, a problem of the graft-versus-host disease (GVHD) in transplantation of a xenogeneic cell, a problem of off-target toxicity, etc.

The immune cell of the present invention can expresses a first chimeric antigen receptor and a second chimeric antigen receptor containing the antigen-binding site that specifically binds to a cancer cell-specific antigen, on a surface of the immune cell, wherein the first chimeric antigen receptor comprises the intracellular domain of the receptor containing a death domain, such as p75^{NTR}, as the intracellular signaling domain, and the antigen-binding site that specifically binds to an antigen expressed in both of the normal and cancer cells. In this case, if only the normal cell is present, only the first chimeric antigen receptor out of the dual chimeric antigen receptors can recognize and bind to the antigen on the surface of the normal cell, whereby, as described above, cytotoxicity of the immune cell can be reduced or apoptosis of the immune cell can be promoted so that an immune response cannot be caused to the normal cell. On the other hand, in case the cancer cell exists, both of an antigen that can be recognized by the first chimeric antigen receptor and an antigen that can be recognized by the second chimeric antigen receptor may be expressed on the surface of the cancer cell, whereby signal transmission occurs in the two chimeric antigen receptors all. In case the signal transmission occurs in both the first chimeric antigen receptor and the second chimeric antigen receptor, cytotoxicity of the immune cell can be further enhanced compared to activation of the immune cell through the signal transmission of the second chimeric antigen receptor alone. Therefore, the immune cell of the present invention can exhibit stability for the normal cell and exhibit increased cytotoxicity compared to the cytotoxicity against the cancer cell shown by the existing chimeric antigen receptor, thereby improving an efficacy of attacking and treating the cancer cell.

In a specific embodiment of the present invention, as a result of confirming cytotoxicity against a breast cancer cell expressing EGFR and HER2 or a breast cancer cell expressing both EGFR and EphA2 by using the natural killer cells expressing all of the first chimeric antigen receptor capable of specifically binding to EGFR expressed in both of the normal cell and the cancer cell and the second chimeric antigen receptor capable of specifically binding to HER2 or EphA2 specifically expressed in the cancer cell, it could be confirmed that the cytotoxicity was higher in the natural killer cell dually expressing the first and second chimeric antigen receptors compared to the natural killer cell expressing only the second chimeric antigen receptor. Therefore, it can be confirmed that the immune cell of the present invention can be very useful for treating the cancer.

Meanwhile, the immune cell of the present invention may express, on its surface, a plurality of additional chimeric antigen receptors, for example, a third, fourth, fifth chimeric antigen receptors, etc., in addition to the above-described first and second chimeric antigen receptors. The plurality of additional chimeric antigen receptors may specifically bind to a completely different type of antigens than the first chimeric antigen receptor or the second chimeric antigen receptor, and the plurality of chimeric antigen receptors may also bind specifically to different types of the antigens, respectively. In case the plurality of additional chimeric antigen receptors as described above are expressed on the surface of the immune cell, the immune cell of the present invention can target a plurality of antigens simultaneously. Ultimately, the immune cell of the present invention can improve cytotoxicity of the immune cell due to the second, third, fourth chimeric antigen receptors, etc. for the target cells containing specific antigens such as the second, third, fourth chimeric antigen receptors, etc., by simultaneously expressing the second, third, fourth chimeric antigen receptors, etc. together with the first chimeric antigen receptor which contains the intracellular domain of the receptor containing the death domain, such as p75^{NTR}, as the intracellular signaling domain, thereby being able to further expand a range of the target cells to which the immune cell of the present invention can exhibit cytotoxicity.

### 3. Use of the immune cell of the present invention for treatment of cancer

Another aspect of the present invention provides a pharmaceutical composition for treating a cancer containing an immune cell.

Since the immune cell, the chimeric antigen receptor expressed therefrom, etc. are the same as those described in "1. Novel chimeric antigen receptor (CAR), and polynucleotide and expression vector for expressing same" and "2. Immune cell expressing dual chimeric antigen receptor (Dual-CAR)", the description therefor is omitted to avoid repeated description.

In the present invention, the term "cancer" and "tumor" are used in the same meaning with each other, and typically refer to or mean a physiological condition in a mammal characterized by unregulated cell growth/proliferation.

A cancer or a carcinoma that can be treated by the composition of the present invention is not particularly limited and includes a solid cancer and a hematological cancer in all. For example, the above cancer may be at least one selected from the group consisting of a lung cancer, a stomach cancer, an ovarian cancer, a cervical cancer, a breast cancer, a pancreatic cancer, a colon cancer, a rectal cancer, an esophageal cancer, a skin cancer, a thyroid cancer, a kidney cancer, a liver cancer, a head and neck cancer, a bladder cancer, a prostate cancer, a blood cancer, a multiple myeloma, an acute myeloid leukemia, a malignant lymphoma, a thymic cancer, an osteosarcoma, a fibrous tumor, and a brain cancer, but is not limited thereto. Any cancer cell containing an antigen that can be recognized by the second chimeric antigen receptor can be applied in the present invention without limitation.

In the present invention, the term "treatment" means inhibiting development of a cancer and alleviating or eliminating a symptom.

The pharmaceutical composition may contain 1 to 10 times, 2 to 10 times, or 5 to 8 times the number of immune cells compared to the number of tumor cells of the subject to be treated, but is not limited thereto.

In addition to the pharmaceutical composition, the composition of the present invention may be in the form of a quasi-drug composition, a health food composition, etc.

The composition of the present invention for treating a cancer may further comprise a pharmaceutically acceptable carrier thereof. The 'pharmaceutically acceptable' means that the subject to be applied (prescribed) does not have toxicity beyond compliance without inhibiting the activity of active ingredients, and the 'carrier' is defined as a compound that can be easily added into a cell or a tissue.

The pharmaceutical composition of the present invention may be administered alone or in mixture with any convenient carrier, etc., and a formulation to be administered may be a single dosage or repeated dosage form. The pharmaceutical composition may be a solid preparation or a liquid preparation. The solid preparation include, but is not limited to, a powder, a granule, a tablet, a capsule, a suppository, etc. The solid preparation may contain, but are not limited to, a carrier, a flavoring agent, a binder, a preservative, a disintegrant, a lubricant, a filler, etc. The liquid preparation include, but are not limited to, a solution such as water and propylene glycol solution, a suspension, and an emulsion, and may be made by adding an appropriate colorant, flavoring agent, stabilizer, viscous agent, etc. For example, the powder may be prepared by simply mixing a trihydroxy derivative of a polyunsaturated fatty acid, which is an active ingredient of the present invention, with a suitable pharmaceutically acceptable carrier such as a lactose, a starch, or a microcrystalline cellulose. The granule may be prepared by mixing the trihydroxy derivative of the polyunsaturated fatty acid of the present invention, a pharmaceutically acceptable carrier, and a suitable pharmaceutically acceptable binder such as polyvinylpyrrolidone and hydroxypropyl cellulose, followed by carrying out a wet granulation process using a solvent such as water, ethanol or isopropanol, or a dry granulation process using a compression force. Also, the tablet may be made by mixing the granule with a suitable pharmaceutically acceptable lubricant such as magnesium stearate followed by compressing the mixture into the tablet using a tablet press.

The pharmaceutical composition may be administered in the form of an oral agent, an injectable agent (e.g., intramuscular injection, intraperitoneal injection, intravenous injection, infusion, subcutaneous injection, implant), an inhalation agent, a nasal administration agent, a vaginal agent, a rectal agent, a sublingual agent, a transdermal agent, a topical agent, etc., depending on the diseases to be treated and a condition of the subject, but is not limited thereto. Depending on the route of administration, it may be formulated in an appropriate dosage unit form containing commonly used, non-toxic, pharmaceutically acceptable carrier, excipient, and vehicle.

The pharmaceutical composition may be administered in a daily dosage of about 0.0001 mg/kg to about 10 g/kg and may be administered in a daily dosage of about 0.001 mg/kg to about 1 g/kg. However, the dosage may vary depending on a degree of purification of the mixture, a patient's condition (age, gender, weight, etc.), and severity of the condition being treated. If necessary, for convenience, the total daily dosage may be administered in divided doses several times throughout a day.

Hereinafter, the present invention will be described in detail through Examples.

However, the following Examples are intended to illustrate the present invention specifically, and the present invention is not limited by the following Examples.

### [Example 1]

### Design of chimeric antigen receptor comprising intracellular domain of the receptor containing death domain

A chimeric antigen receptor (CAR) may comprise an extracellular domain (ectodomain), a transmembrane domain, and an intracellular signaling domain (endodomain). The chimeric antigen receptor of the present invention was designed to include the intracellular domain of p75^{NTR}, one of the receptors containing a death domain, as the intracellular signaling domain.

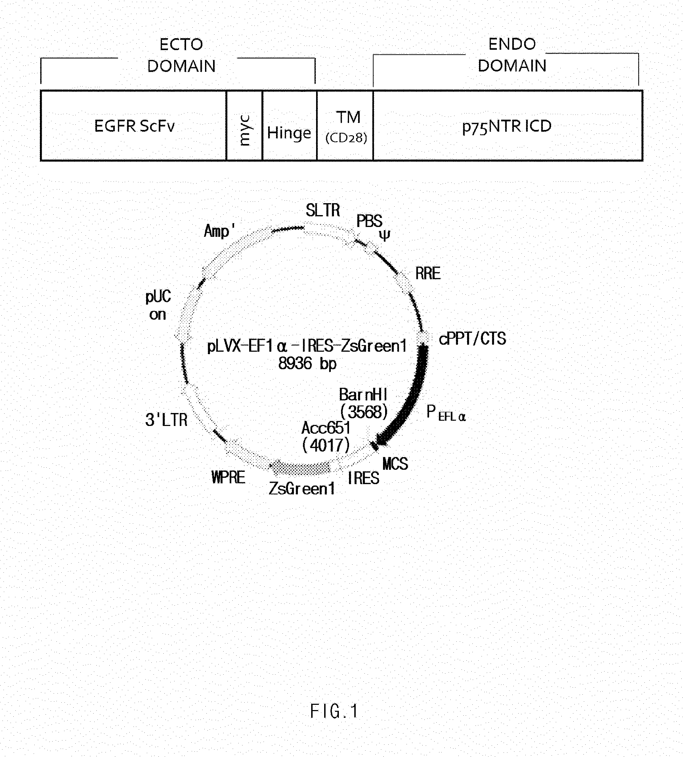

The extracellular domain contains an antigen-binding site, and the chimeric antigen receptor of the present invention can be prepared regardless of a type of the antigen or a type of the antigen-binding site. This Example employed a single chain variable fragment (scFv) of an antibody that can specifically bind to EGFR. It was constructed such that using CD28 as the transmembrane domain, the scFv for EGFR and the CD28 were linked to Myc and a hinge domain, and the intracellular domain of p75^{NTR} was linked to the transmembrane domain, and thus a gene construct containing a base sequence of SEQ ID NO: 13 encoding the receptor was inserted into a pLVX-EF1a-IRES-zsGREEN vector (FIG. 1).

### [Example 2]

### Preparation of immune cell expressing dual chimeric antigen receptor (dual CAR) on its surface

### [2-1] Preparation of immune cell co-expressing CAR containing intracellular domain of α-Cot-CAR and p75^{NTR}

An immune cell capable of expressing on its surface the chimeric antigen receptor of the present invention designed by Example 1 was prepared. In this case, the vector designed by Example 1 was introduced into a α-Cot-CAR NK92 cell (M2), which is a natural killer cell (NK cell) expressing an anti-cotinine chimeric antigen receptor, and thus a Dual-CAR NK92 cell 1, the natural killer cell capable of expressing two types of the chimeric antigen receptors, was prepared.

The α-Cot-CAR NK92 cell is the natural killer cell that can be produced and/or obtained according to the method described in Korean Patent No. 2,122,546, and has the characteristic of expressing on its surface the chimeric antigen receptor containing an scFv capable of specifically binding to a cotinine as an extracellular domain. Specifically, the α-Cot-CAR NK92 cell was separated into a single cell (M2) through an automated high-speed flow cytometer (FacsAria fusion), and a vector encoding the chimeric antigen receptor of the present invention designed by Example 1 was introduced into the α-Cot-CAR NK92 cell using lentivirus. Accordingly, in the case of the natural killer cell into which the vector has been successfully introduced, not only the previously expressed anti-cotinine chimeric antigen receptor but also the chimeric antigen receptor of the present invention containing the intracellular domain of p75^{NTR} can be expressed on a surface of the cell.

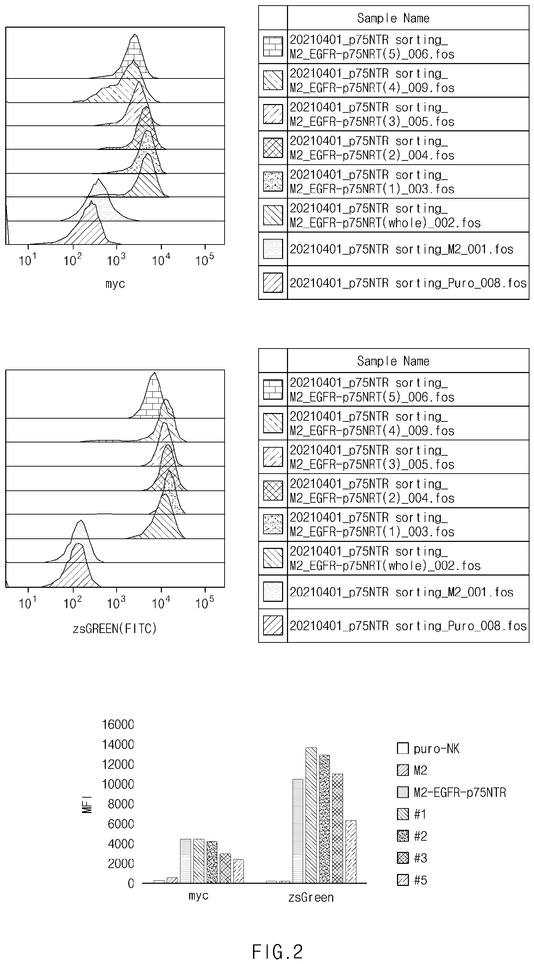

After introducing the vector encoding the chimeric antigen receptor of the present invention, it was identified whether or not the chimeric antigen receptor was expressed from the natural killer cell line. Specifically, in order to detect a tag protein Myc contained in the chimeric antigen receptor, the cell line was stained using a fluorescent Myc antibody, and expression on a surface of the cell was confirmed through a flow cytometry. In addition, expression of the EGFR-p75^{NTR} could be confirmed by measuring a fluorescence of zsGreen expressed from the inserted vector through the flow cytometry, which results in showing that the two chimeric antigen receptors were successfully expressed (FIG. 2).

### [2-2] Preparation of immune cell co-expressing CAR containing intracellular domain of α-EphA2-CAR and p75^{NTR}

Additionally, other immune cell capable of expressing the chimeric antigen receptor of the present invention designed by Example 1on a surface of the immune cell was prepared.

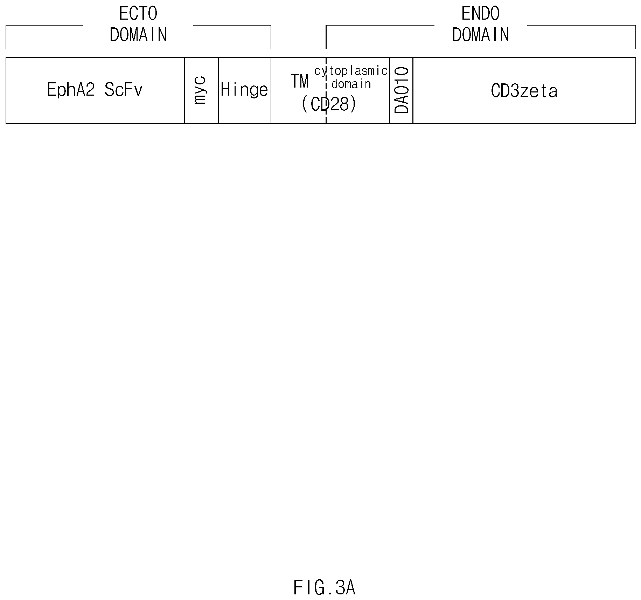

First, a chimeric antigen receptor (CAR) to be introduced into a natural killer cell was designed with an extracellular domain containing an anti-EphA2 single chain variable fragment (scFv) as an antigen-binding site. Specifically, the chimeric antigen receptor of the present invention was designed to have an intracellular signaling domain of the chimeric antigen receptor, which is classified as the so-called third-generation CAR, by linking the extracellular domain containing the scFv as the antigen-binding site to a Myc and a hinge domain using CD28 as the transmembrane domain, and linking to the transmembrane domain with addition of CD3-zeta as the intracellular signaling domain and CD28 DAP10 as a co-stimulatory molecule (FIG. 3A). Then, the base sequence of a gene construct encoding the chimeric antigen receptor was inserted into a lentiviral vector and transformed into a HEK293T-cell along with a viral packaging vector (pMDLG/RRE, pRSV/REV, VSVG). From it, the lentivirus expressing EphA2-CAR1 was obtained, concentrated using an ultrahigh-speed centrifuge, and then infected with a natural killer such that a multiplicity of infection (MOI) became 30 by a spinoculation method (360 g, 90 min, RT). The natural killer cell infected as above was cultured under 5% CO₂ condition at 37 °C for 5 hours, followed by being replaced with a fresh medium, and treated with puromycin in a concentration of 3 ug/ml after 3 days so as to select a properly infected natural killer cell. The culture was continuously performed. The uninfected natural killer cell as a control group was also treated with the puromycin, and the culture was continuously performed until all the natural killer cells in the control group were killed by the puromycin, using a puromycin-treated medium. At the point when all the natural killer cells in the control group were killed, the infected natural killer cells were selected to perform the experiment. After separating the α-EphA2-CAR NK92 cell selected as described above into a single cell through an automated high-speed flow cytometer (79-14), a Dual-CAR NK92 cell 2, the natural killer cell, which can express on the cell surface both the previously expressed anti-EphA2 chimeric antigen receptor and the chimeric antigen receptor of the present invention containing the intracellular domain of p75^{NTR}, was prepared by introducing the vector designed by Example 1 in the same method as that of Example [2-1].

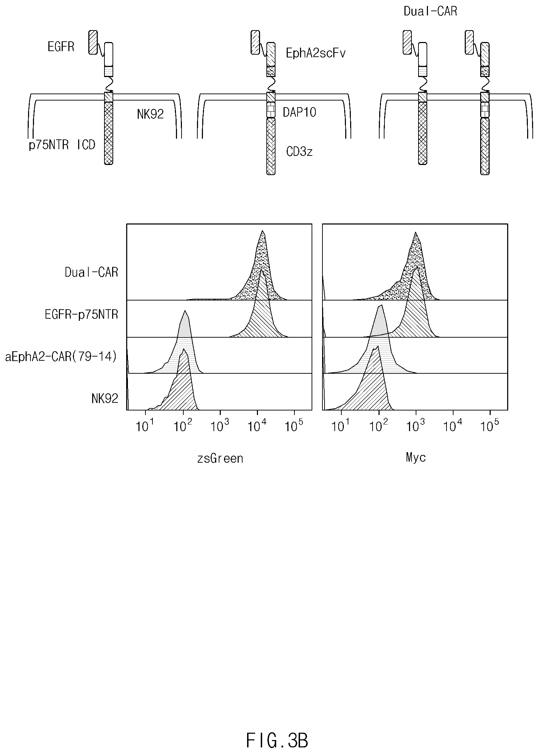

As a result of confirming whether or not the chimeric antigen receptor was expressed in the Dual-CAR NK92 cell 2 prepared above by the same method as that of Example [2-1], it could be identified that the two types of chimeric antigen receptors were successfully expressed (FIG. 3B).

### [Example 3]

### Confirmation of function of p75^{NTR} signal in natural killer cell when only antigen expressed in both cancer cell and normal cell is present

The natural killer cell prepared by Example [2-1] has a characteristic of expressing, on its surface, a chimeric antigen receptor (the first CAR) containing the intracellular domain of p75^{NTR} as a signaling domain and a chimeric antigen receptor (the second CAR) containing an antigen-binding site that can specifically bind to a cotinine. In this case, the first CAR contains a single chain variable fragment (scFv) of an antibody that can recognize and bind to EGFR expressed in both the normal cell and the cancer cell, so that signal transmittance can occur within a natural killer cell by the normal cell or the cancer cell expressing EGFR on its surface.

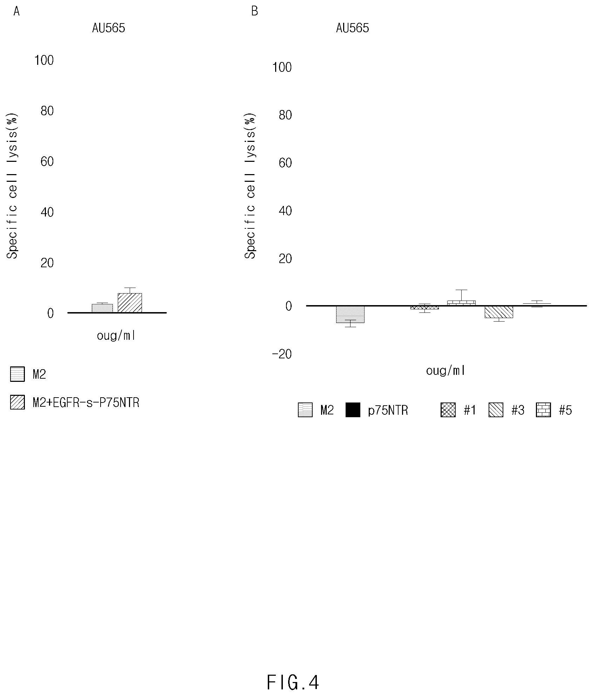

Accordingly, in order to identify activity of the natural killer cell in case only the chimeric antigen receptor (the second CAR) of the present invention containing the intracellular domain of p75^{NTR} is activated alone, AU565, a breast cancer cell expressing EGFR on its surface was used as a target cell, was stained with calcein under the condition that the AU565 and the cotinine conjugate is not added to prevent signal transmittance of the second CAR, and was co-cultured with the natural killer cell of Example [2-1] (Dual-CAR NK92) or a α-Cot-CAR NK92 cell (M2) at a ratio of 1:3 (target cell: natural killer cell) for 4 hours. Cytotoxicity of the natural killer cell was confirmed by an amount of calcein secreted in a supernatant after 4 hours of the co-culture. As a result, the α-Cot-CAR NK92 cell (M2) failed to bind to EGFR and did not generated the signal transmittance due to the absence of the cotinine conjugate, which resulted in not also displaying the cytotoxic activity of the natural killer cell. Likewise, the natural killer cell (Dual-CAR NK92) of Example [2-1] expressing the chimeric antigen receptor containing the intracellular domain of p75^{NTR} also did not show the cytotoxic activity of the natural killer cell (FIG. 4).

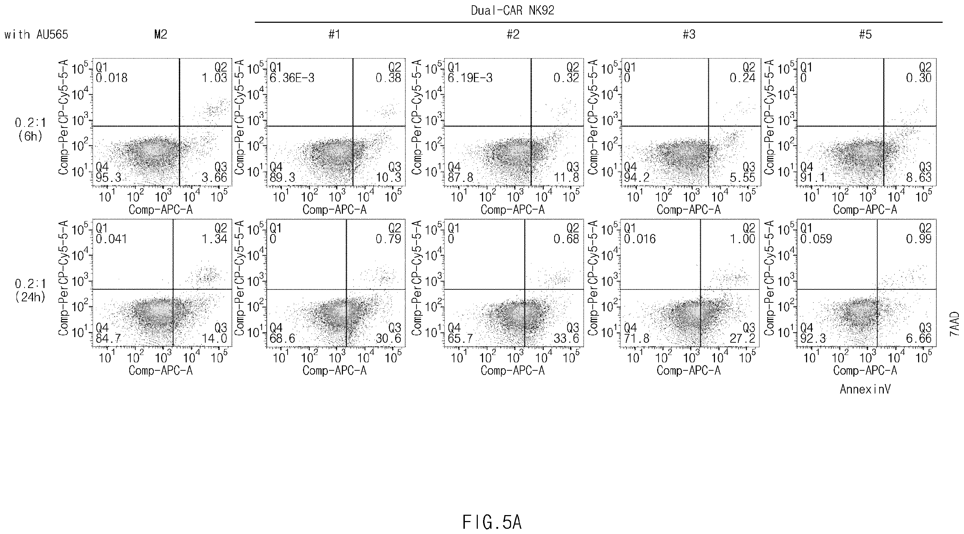

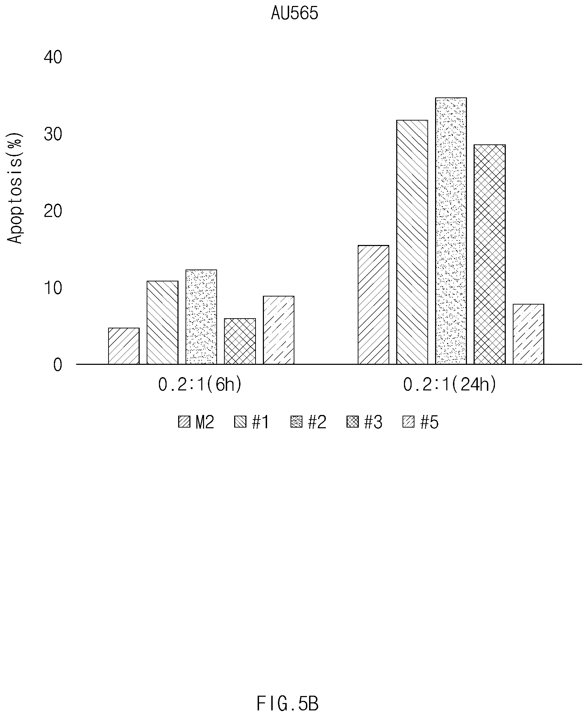

In addition, after culturing the natural killer cell with the AU565 cell, a degree of apoptosis of the natural killer cell was confirmed at the point when 6 hours and 24 hours lapsed, respectively. The apoptosis was measured after co-culturing the AU565 cell with the natural killer cell (Dual-CAR NK92) of Example [2-1] or the M2 cell at a ratio of 1:0.2 (target cell: natural killer cell) for 6 or 24 hours, respectively. After co-culturing each of the cells, the cells were stained with annexinV and 7AAD, and then a degree of the apoptosis was confirmed through a flow cytometry. In this case, the M2 cell was stained with an antibody expressing a fluorescence of CD56-FITC, and the natural killer cell of Example [2-1] was distinguished from the target cell, AU565, using zsGreen which is expressed by itself, so that only the natural killed cell was compartmentalized to confirm a degree of the apoptosis. As a result, when the natural killer cell (Dual-CAR NK92) of Example [2-1] was exposed to the AU565 cell expressing EGFR for a long time, it was confirmed that the apoptosis of the natural killer cell was increased compared to the α-Cot-CAR NK92 cell (M2) (FIG. 5).

Considering the above results comprehensively, when the chimeric antigen receptor containing the intracellular domain of p75^{NTR} is expressed on a surface of the natural killer cell and the signal transmittance is activated only by the chimeric antigen receptor alone, it could be confirmed that the cytotoxic activity of the natural killer cell was not observed, and rather that the apoptosis of the natural killer cell was induced when exposed to the antigen for a long period of time, thereby making the natural killer cell unable to function properly. Since the chimeric antigen receptor of the present invention containing the intracellular domain of p75^{NTR} was designed to recognize and bind to an antigen expressed in both of the cancer cell and the normal cell, if only the normal cell is present, the natural killer cell is not toxic to the normal cell and induces the apoptosis. Therefore, it could be confirmed that the natural killer cell expressing the chimeric antigen receptor of the present invention on its surface has the characteristic of not attacking the normal cell.

### [Example 4]

### Confirmation of cytotoxicity of natural killer cell expressing dual CAR in the presence of cancer cell-specific antigen

In addition to confirming from Example 3 that the cytotoxicity of the natural killer cell was reduced and the apoptosis was increased when single signal transmittance occurred by the chimeric antigen receptor containing the intracellular domain of p75^{NTR}, it was examined how activity of the natural killer cell changed when signal transmittance all occurred by the dual CAR of the natural killer cell due to the presence of a cancer cell-specific antigen.

Specifically, a AU565 cell, the breast cancer cell expressing both EGFR and HER2, was used as a target cell to co-culture a α-Cot-CAR NK92 cell (M2) and the natural killer cell (Dual-CAR NK92) of Example [2-1], respectively. In this case, a HER2-cotinine (HER2-cot) conjugate was used together to recognize HER2 expressed from the AU565 cell, so that it can be recognized and bound by the chimeric antigen receptor expressed on the surface of the α-Cot-CAR NK92 cell (M2) and the natural killer cell (Dual-CAR NK92) of Example [2-1] (see Korean Patent No. 2,122,546).

The cytotoxicity of the natural killer cell co-cultured with the AU565 cell was confirmed in the same method as that of Example 3.

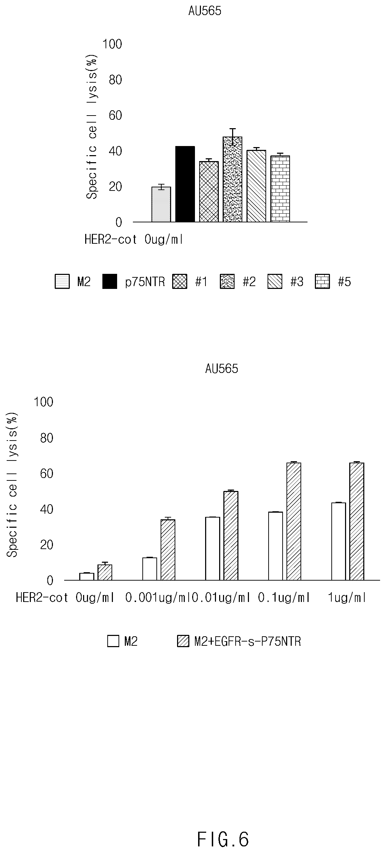

As a result, it could be confirmed that the cytotoxicity of the natural killer cell (Dual-CAR NK92) of Example [2-1] increased significantly in the presence of the HER2-cotinine conjugate, compared to the cytotoxicity of the α-Cot-CAR NK92 cell (M2) expressing only the anti-cotinine chimeric antigen receptor. The results confirmed for each single cell also showed higher cytotoxicity in all Dual-CAR NK92 cells, compared to the α-Cot-CAR NK92 cell (M2) (FIG. 6).

The natural killer cell expressing an anti-cotinine chimeric antigen receptor recognizes HER2 on a surface of the cancer cell through the HER2-cotinine conjugate, which resulted in having cytotoxicity through signal transmission and increasing activity to attack the cancer cell. However, from results of the above experiment, it can be seen that, in case the chimeric antigen receptor containing the intracellular domain of p75^{NTR} is dually expressed to generate signal transmittance by EGFR on a surface of the cancer cell, the cytotoxicity of the natural killer cell is further increased.

### [Example 5]

### Additional verification of cytotoxicity of dual CAR-expressed natural killer cell

It was verified once again that the apoptosis of the natural killer cell itself is increased when signal transmission occurs alone using only the chimeric antigen receptor containing the intracellular domain of p75^{NTR} identified from Example 3, and that the cytotoxicity of the natural killer cell is improved when the cancer-specific antigen identified from Example 4 exists together to generate signal transmission in both the CARs, by way of using the natural killer cell (α-EphA2-CAR NK92 cell) expressing the CAR with a different type of the extracellular domain, rather than using the natural killer cell expressing the α-Cot-CAR.

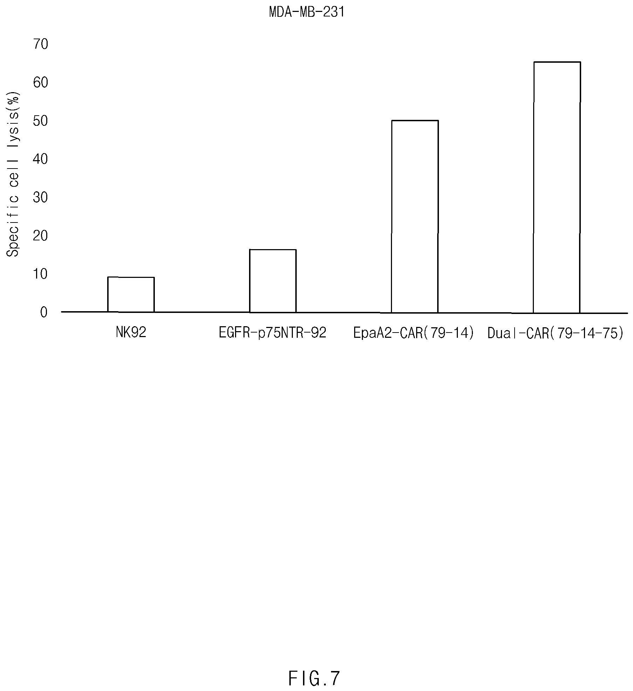

Specifically, i) a NK92 cell, ii) an EGFR-p75^{NTR} NK92 cell produced by introducing a vector encoding the chimeric antigen receptor of the present invention designed by Example 1 into the NK92 cell in the same method as that of Example [2-1], iii) a α-EphA2-CAR NK92 cell (79-14) produced by Example [2-2], and iv) a Dual-CAR NK92 cell 2 (79-14-75) produced by Example [2-2] were co-cultured, respectively, by way of using a MDA-MB-231 cell, which is the breast cancer cell expressing both EGFR and EphA2 as a target cell, and were tested for the cytotoxicity of each natural killer cell in the same method as that of Example 3. As a result, it was identified that a comparison of the cellular activity of i) the NK92 cell and ii) the EGFR-p75^{NTR} NK92 cell exhibited no increase in the cytotoxicity in case signal transmission occurred alone by using only the chimeric antigen receptor containing the intracellular domain of p75^{NTR}, and that the cytotoxicity of iv) the Dual-CAR NK92 cell 2 (79-14-75) was much improved compared to that of iii) the α-Epha2-CAR NK92 cell (79-14), which express only the anti-EphA2 chimeric antigen receptor (FIG. 7).

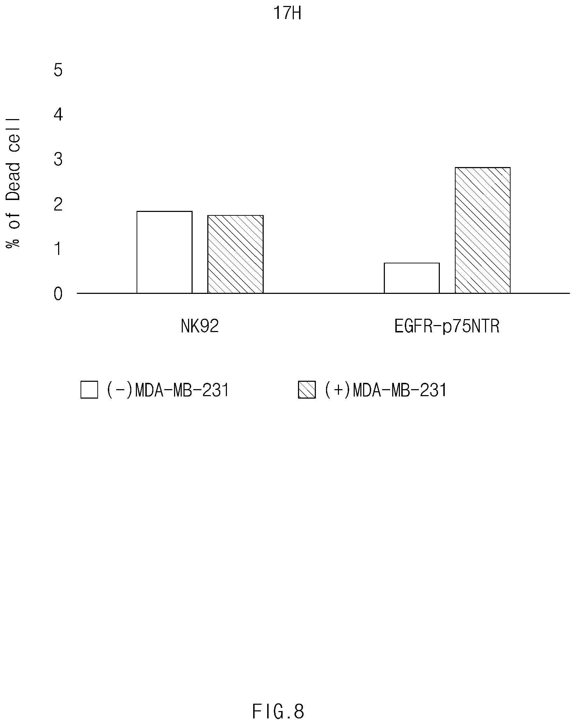

Also, the above natural cells were cultured with a MDA-MB-231 cell as in Example 3, and tested for their apoptosis at the point when 17 hours lapsed. As a result, it was identified that i) the NK92 cell after co-cultured with the target cell did not show increase in its apoptosis compared to that before co-cultured, but ii) the EGFR-p75^{NTR} NK92 cell showed increase in the apoptosis after co-cultured with the target cell (FIG. 8).

The above results are the same as those of the Dual-CAR NK92 cell 1 of the α-Cot-CAR NK92 cell. It can be seen from these results that the Dual-CAR using the chimeric antigen receptor of the present invention containing the intracellular domain of p75^{NTR} as the signaling domain shows the same efficacy even for the antigens other than the cotinine.

### [Example 6]

### Confirmation of effect of apoptosis by death domain of intracellular domain of P75^{NTR}

An intracellular domain of P75^{NTR} contains a death domain, and this domain is known to be involved in apoptosis. It was confirmed through Examples 3 and 5 of the present invention that the apoptosis was increased by signal transmission alone of p75^{NTR}. It was investigated whether this effect was actually exerted by the death domain present in the intracellular domain of p75^{NTR}.

Specifically, in order to identify function of the death domain, a chimeric antigen receptor (p75^{NTR} DDD) was designed in which the death domain of the intracellular domain of p75^{NTR} was removed from the chimeric antigen receptor designed by Example 1, and a gene construct encoding it was inserted into a pBlueScript SK(-) vector (FIG. 9). Apart from this, a gene construct encoding the chimeric antigen receptor designed by Example 1 was also inserted into the pBlueScript SK(-) vector, and mRNA of the EGFR-p75^{NTR} CAR and the EGFR-p75^{NTR} DDD was prepared through in vitro transcription from each of these two vectors. The mRNA prepared as described above was transformed into a α-Cot-CAR NK92 cell (M2), and then after 17 hours lapsed, expression of the introduced chimeric antigen receptor was confirmed by detecting a tag protein Myc in the same method as that of Example 2. As a result, it was confirmed that the two chimeric antigen receptors from which the p75^{NTR} and the death domain were removed were successfully expressed (FIG. 10).

The cytotoxicity was identified by co-culturing a target cell, AU565 cell, and each Dual-CAR NK92 cell at a ratio of 1:1, 1:0.5, and 1:0.25 (target cell: natural killer cell) in the same method as that of Example 4, using the Dual-CAR NK92 cell expressing each chimeric antigen receptor as described above. As a result, regardless of whether the death domain was removed, the Dual-CAR NK92 cell showed higher cytotoxicity than the α-Cot-CARNK92 cell. In case only the chimeric antigen receptor containing the intracellular domain of p75^{NTR} was activated alone without adding the HER2-cotinine (HER2-cot) conjugate, it was confirmed that the cytotoxicity did not increase regardless of whether the death domain was removed (FIG. 11). It can be seen that this is the same result as that of Examples 3 to 5.

Meanwhile, since the death domain is known to affect apoptosis, a degree of the apoptosis of the Dual-CAR NK92 cell was identified at the point when 24 hours lapsed after co-culturing the Dual-CAR NK92 cell with the target-cell, AU565. Identifying a degree of the apoptosis was performed by co-culturing the Dual-CAR NK92 cell and the target cell at a ratio of 0.5:1 in the same method as that of Example 3 without adding the HER2-cotinine (HER2-cot) conjugate so that only the intracellular signal of p75^{NTR} is transmitted. As a result, it was found that, while a degree of the apoptosis for each Dual-CAR NK92 cell which was not co-cultured with the AU565 was similar in all, when co-cultured with the AU565, the Dual-CAR NK92 cell expressing the chimeric antigen receptor containing the intracellular domain of p75^{NTR} showed increase in the apoptosis compared to the α-Cot-CAR NK92 cell lacking the intracellular domain of p75^{NTR}. Also, the Dual-CAR NK92 cell expressing the chimeric antigen receptor containing the death domain removed was found to have a reduced degree of the apoptosis (FIG. 12). From the above results, it can be seen that the apoptosis is induced when the intracellular signaling is activated solely by the p75^{NTR}, and that induction of this apoptosis is caused by the death domain present in the intracellular domain of p75^{NTR}.

Therefore, it could be confirmed that the chimeric antigen receptor of the present invention comprising as the intracellular signaling domain the intracellular domain of the receptor containing the death domain, such as p75^{NTR}, has no or little cytotoxicity against the normal cell and induces the apoptosis of the natural killer cell, so that the chimeric antigen receptor can ensure stability for the normal cell as well as exhibit more powerful cytotoxicity against the cancer cell than the existing technology using the single chimeric antigen receptor. Accordingly, it was confirmed that the chimeric antigen receptor and the natural killer cell can be usefully used as a cancer therapy having excellent efficacy.

As described above, the present invention has been described in detail by way of only Examples of the specification, but it is obvious to those skilled in the art that various changes and modifications are possible within the technical scope of the present invention, and it is also natural that such changes and modifications fall within the scope of the appended claims.

## Claims

1. A chimeric antigen receptor (CAR) comprising:
an extracellular domain (extracellular binding domain) containing an antigen-binding site;
a transmembrane domain; and
an intracellular signaling domain comprising an intracellular domain of the receptor containing a death domain.

2. The chimeric antigen receptor according to claim 1,
wherein the receptor containing the death domain is any one selected from the group consisting of Fas, TNFRI, p75^{NTR}, DR3, DR4, DR5, EDAR and DR6.

3. The chimeric antigen receptor according to claim 1,
wherein the antigen-binding site specifically binds to an antigen expressed in both of a cancer cell and a normal cell.

4. The chimeric antigen receptor according to claim 1,
wherein the antigen-binding site specifically binds to EGFR.

5. The chimeric antigen receptor according to claim 1,
wherein the antigen-binding site is a single chain variable fragment (scFv) of an antibody.

6. The chimeric antigen receptor according to claim 1,
wherein the extracellular domain further contains at least one selected from the group consisting of a hinge domain and a spacer domain.

7. The chimeric antigen receptor according to claim 5,
wherein the hinge domain or the spacer domain is at least one selected from the group consisting of a Myc epitope, a CD8 hinge domain, and a Fc.

8. The chimeric antigen receptor according to claim 1,
wherein the transmembrane domain is any one selected from the group consisting of an alpha (α), beta (β) or zeta (ζ) chain of a T-cell receptor (TCR), CD28, CD3 epsilon (ε), CD45, CD4, CD5, CD8, CD9, CD16, CD22, CD33, CD37, CD64, CD80, CD86, CD134, CD137, and CD154.

9. A polynucleotide comprising a base sequence encoding the chimeric antigen receptor according to any one of claims 1 to 8.

10. An expression vector comprising the polynucleotide of claim 9.

11. An immune cell of expressing the chimeric antigen receptor according to any one of claims 1 to 8 as a first chimeric antigen receptor on a surface of the immune cell; and
expressing, on a surface of the immune cell, a second chimeric antigen receptor comprising an extracellular domain containing an antigen-binding site that specifically binds to an antigen expressed in a cancer cell, a transmembrane domain, and an intracellular signaling domain.

12. The immune cell according to claim 11,
wherein the immune cell is any one selected from the group consisting of a natural killer cell (NK cell), a T-cell, a natural killer T-cell (NKT-cell), a cytokine-induced killer cell (CIK), a macrophage, and a dendritic cell.

13. The immune cell according to claim 11,
wherein the immune cell further expresses, on its surface, at least one chimeric antigen receptor comprising an extracellular domain containing an antigen-binding site that specifically binds to an antigen different from the first chimeric antigen receptor and the second chimeric antigen receptor, a transmembrane domain, and an intracellular signaling domain.

14. The immune cell according to claim 11,
wherein the antigen-binding site of the second chimeric antigen receptor specifically binds to any one selected from the group consisting of HER2, EphA2, ErbB3, IL-13Rα2, DLK1, B7H3, PD-L1, GPC3, CEACAM6, and CD5.

15. A pharmaceutical composition for treating a cancer, comprising the immune cell of claim 11.

16. The pharmaceutical composition according to claim 15,
wherein the cancers is at least one selected from the group consisting of a lung cancer, a stomach cancer, an ovarian cancer, a cervical cancer, a breast cancer, a pancreatic cancer, a colon cancer, a rectal cancer, an esophageal cancer, a skin cancer, a thyroid cancer, a kidney cancer, a liver cancer, a head and neck cancer, a bladder cancer, a prostate cancer, a blood cancer, a multiple myeloma, an acute myeloid leukemia, a malignant lymphoma, a thymic cancer, an osteosarcoma, a fibrous tumor, and a brain cancer.
